**Europäisches Patentamt**

**European Patent Office**

**Office européen des brevets**

(19)

(11)  **EP 1 376 124 A2**

(12)  **EUROPEAN PATENT APPLICATION**

(43) Date of publication:
**02.01.2004 Bulletin 2004/01**

(51) Int Cl.⁷: **G01N 33/50**, **B01L 3/00**

(21) Application number: **03076980.6**

(22) Date of filing: **26.06.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **27.06.2002 US 392275 P**

(71) Applicant: **Corning Incorporated
Corning, New York 14831 (US)**

(72) Inventors:
• **Fang, Ye
Painted Post, NY 14870 (US)**

• **Frutos, Anthony Glenn
Painted Post, NY 14870 (US)**
• **Lahiri, Joydeep
Painted Post, NY 14870 (US)**

(74) Representative: **Poole, Michael John
Corning Limited
Patents & Licensing Department
Quantum House,
Maylands Avenue
Hemel Hempstead, Herts. HP2 7DE (GB)**

(54)  **Toxin detection and compound screening using biological membrane microarrays**

(57)     A microarray containing components of cell membranes and a high-throughput method for using the microarray to detect toxins and screen for other biological or chemical compounds that may block the binding of toxin molecules to their target binding sites is provided. The microarray according to the present invention provides an attractive platform for efficient study of fundamental aspects of molecular recognition at the cell surface. Specific binding pattern of a given toxin to a set of different biological membrane probes in the microarray can be employed as a "signature" to identify and detect the presence of a toxin in a sample.

**EP 1 376 124 A2**

## Description

CLAIM OF PRIORITY

The present application claims benefit of priority from U.S. Provisional Application No. 60/392,275, filed 27th June 2002, the content of which is incorporated herein by reference.

FIELD OF THE INVENTION

The present invention pertains to detection of biological and chemical molecules. In particular, the invention uses biological membrane microarrays for detecting and identifying toxins, and screening compounds that can inhibit toxin binding.

BACKGROUND

Many prokaryotic and eukaryotic animals and plants produce toxins, which can have harmful and sometimes lethal effects on other living organisms. All animal toxins target a cell surface protein that involves in an essential cell function. Nearly all of these toxins are ligands of ion channels, which regulate rapid transport activity into or out of the cell. When toxin molecules bind to ion channels, the ion channels are inactivated, and consequently the toxin interferes with the biochemical mechanisms for important specific cellular functions, such as neurological or muscular functions. Some toxins bind to proteins on the cell surface during pathogenesis - examples include the binding of diphtheria toxin *(corynebacterium diphtheriae)* and anthrax toxin *(bacillus anthracis).* Various kinds of toxins can target a specific function. For example, just to name a few, tetrodotoxin, saxitoxin, bactrachotoxin, grayanotoxin, veratridine, actonitine, scorpion and sea anemone venom can attack different sites of sodium channels and block their function; and, some other toxins including apamin and related peptides, scorpion charybdotoxins, dendrotoxins, hanatoxins, sea anemone toxins target specifically potassium channels. Other toxins such as hololena or calcicludine target calcium channels, while toxins such as bungarotoxin and conantokins target, respectively, target nicotinic acetylcholine receptors and glutamate receptors.

Various other molecules on the surface of the host cell are targets for toxin binding. For example, cholesterol is the target molecule for bacteria from the genera *streptococcus, bacillus, clostridium,* and *listeria.* A large number of bacterial toxins target carbohydrate derivatized lipids on the cell surface, often with high specificity. These lipids, glycosylated derivatives of ceramides referred to as sphingoglycolipids, can be classified into cerebrocides (ceramide monosaccharide), sulfatides (ceramide monosaccharide sulfates) and gangliosides (ceramide oligosaccharides).

Moreover, bacteria use a variety of sophisticated strategies for survival and modification of host physiology in order to promote their own multiplication and spread. For that purpose, bacterial toxins use several different mechanisms. A large of group of toxins alter the plasma permeability barrier by inserting and re-organizing into cell membranes, while other toxins act inside the cell. Many of these bacterial toxins acting inside a cell usually consist of two functional domains - the first is involved in binding to the cell membrane (binding domain; termed B) and the second is involved in intracellular enzymatic activities (activating domain; termed A). For example, cholera toxin consists of two domains, pentameric B domain and an A domain. The pentameric B domain specifically binds to the ganglioside-rich domains on a cell surface.

Chemical toxins, either naturally occurring or man-made, are also a concern. Many kinds of chemical toxin molecules also act on cell surface proteins, including G protein-coupled receptors and ion channels. The chemical toxin molecules can specifically bind to these types of receptors to either activate or deactivate them. Depending on the particular function, the over- or under-activation of receptors can cause illness or other serious consequences.

With so many species of toxins, the task of detecting and identifying toxins, heretofore, has been rather slow and cumbersome. At a time when bacterial resistance to antibiotics is on the rise, and biological warfare is a significant concern, the development of multiplexed, bioanalytical platforms for studying toxins and other proteins in near native environments is especially pertinent. A need exists for a method and device, which can be used for high-throughput screening of toxins, as well as screening of compounds that can block the binding of toxins to target sites.

SUMMARY OF THE INVENTION

The present invention provides a high-throughput method for using biological membrane microarrays to identify and detect toxins in a sample and to screen for compounds that can block the binding of toxin molecules to targets. The method comprises several steps: i) providing an array having a plurality of biological membranes associated with a surface of a substrate; ii) contact the array with a sample or sample solution having a target compound; iii) monitoring for binding activity of at least one biological membrane with the target compound, or in other words, detecting a binding event between at least one or more of biological membrane probe microspots associated with the substrate surface with one or more of the target constituents.

The biological membranes contain a toxin-binding moiety. According to an embodiment, the toxin-binding moiety may be a cell-surface protein. The cell-surface protein is an ion channel, such as for a sodium channel, a potassium channel, a calcium channel, an acetylcholine receptor (e.g., nicotinic acetylcholine receptor), a ryanodine receptor, a glutamate receptor, and any combination of ion channels. The cell-surface protein is a receptor, such as G protein-coupled receptors. In another embodiment, the toxin-binding moiety is a natural lipid, a synthetic lipid, or a lipid composition containing a host lipid and a receptor, or a purified receptor. The receptor can be a ganglioside, a ceramide, a cerebroside, a sulfatide, or cholesterol. The method employs labeled toxin or a know compound with an affinity to the toxin molecule or to the receptor site. The toxin to be detected in the sample can be a synthetic or natural toxin derived from animal, including human, plant, infectious tissues, food, or environmental sources.

In another aspect, the present invention also includes an array comprising a plurality of biological membrane probes, containing a toxin-binding moiety. The biological membranes are arranged in distinct microspots associated with a surface of a substrate for use according to the present method. In some cases, a biological membrane or a composition containing biological membranes that do not have toxin-binding receptors also may be included in the array as a negative control.

The target compound can have at least one constituent that is labeled. When the target compound is labeled, the monitoring or detection step comprises detecting for the presence of the label. Detection may be done preferably by imaging based on the fluorescence, phosphorescence, chemiluminescence, or resonance light scattering, which emanate from the bound target molecule. Prior to detection, the substrate can be washed to remove unbound targets. Alternatively, when the target compound has no labeled constituent, the monitoring step comprises detecting directly a physical change due to the binding of the target compound to the biological membranes. Preferably, the change in physical properties at the biological interface is a change in refractive index or electrical impedance. For example, surface plasmon resonance and other optical sensor technologies may be employed.

For uses involving screening for compounds, competitive binding assays may be applied to a sample containing labeled toxin molecules and a known compound, to generate an inhibitor profile. The array of probe microspots can be incubated with labeled and unlabeled target compounds. The binding event between labeled and unlabeled target compound and the probe is determined by measuring a decrease in the signal of the label due to competition between the two types of target compounds for the probe. The compound may inhibit the binding of the toxin in two possible ways. According to one way, the compound may bind to the receptor site, thereby deactivating the receptor site on the cell membrane. Alternatively, the compounds may bind to the toxin molecule itself, rendering the toxin molecule incompatible with cellular receptor sites. The specific binding pattern of a given toxin to a set of different biological membrane probes in the microarray can be used as a "signature" to identify and detect the presence of a toxin in the sample.

According to an embodiment, the invention provides an immobilized membrane array comprising a biological membrane associated with a surface of a substrate coated with an amine-presenting compound. The substrate can be made of or include a glass, ceramic, silicon, metal, non-metal, polymer or plastic surface, and may be configured as a bead, chip, slide, multiwell microplate, or a microcolumn, as described in PCT Publication No. WO 03/022,421, B.L. Webb *et al.,* incorporated herein by reference, and the like. The substrate may be smooth or flat, uneven or porous. The surface can be coated with a material, such as a silane, thiol, disulfide, or a polymer. The silane may present terminal polar moieties, such as hydroxyl, carboxyl, phosphate, sulfonate, or amino groups. The surface may be positively charged and contain amino groups, such as γ-aminopropylsilane. Alternatively, when the substrate comprises a gold or gold-coated surface, the amine presenting compound molecule can be 11-mercaptoundecylamine or a thiol. Alternatively, to create a hydrophilic surface, the amine-presenting compound can be a polymer. The polymer may be a poly-lysine, a polyethyleneimine, or chitosan. Preferably, the immobilized membrane comprises a toxin-binding receptor.

In additional embodiments, the present invention contemplates using the present arrays in biosensor and diagnostic devices.

Other features and advantages of the present method and array device will become evident from the following detailed description. It is understood that both the foregoing general description and the following detailed description and examples are merely representative of the invention, and are intended to provide an overview for understanding the invention as claimed.

BRIEF DESCRIPTION OF THE FIGURES

Figure 1 shows structures of GM1 and GT1b gangliosides.

Figures 2A, B, and C are fluorescence images of microarrays consisting of DLPC (top row), DLPC doped with 4 mol % GM1 (middle row), and DLPC doped with 4 mol % GT1b (bottom row), treated with solutions of toxins. The images correspond to microarrays treated with: (2A) buffer only; (2B) 1nM fluorescein-labeled cholera toxin (B domain; FITC-CTx); and (2C) 2nM fluorescein-labeled tetanus toxin (C fragment; FITC-TTx).

Figure 2D shows histograms of the relative amounts of binding of the labeled cholera and tetanus toxins to the ganglioside microarrays. The data are normalized to the binding signal of FITC-CTx to GM1 and FITC-TTx to GT1b. RFU = relative fluorescence units.

Figures 3A, B, C, D are fluorescence images of microarrays of DLPC (top row), DLPC doped with 4 mol % GM1 (middle row), and DLPC doped with 4 mol % GT1b (bottom row), treated with solutions containing FITC-CTx in the absence and presence of different inhibitors. The images correspond to microarrays treated with: (3A) FITC-CTx (1 nM); (3B) FITC-CTx (1 nM) and unlabeled tetanus toxin (100 nM); (3C) FITC-CTx (1 nM) and unlabeled bungaratoxin (100 nM); and (3D) FITC-CTx (1 nM) and unlabeled cholera toxin (100 nM).

Figures 4A and B are fluorescence images of microarrays. The fluorescence images of (4A) shows the GM1 ganglioside (4 mol % in DLPC) treated with solutions of FITC-CTx at concentrations ranging from 0.031 nM to 2.0 nM. Fluorescence images of (4B) show an identical set of microarrays treated with FITC-CTx at the same concentrations and excess unlabeled cholera toxin (100 nM). These residual signals provide an estimate of the amount of non-specific binding at each concentration of FITC-CTx.

Figure 4C shows graphs of the concentration dependence of the total fluorescence signal (in RFU), the signal due to non-specific binding, and the signal due to specific binding (estimated as the difference in the signals between (4A) and (4B) at each concentration of FITC-CTx).

Figure 5 shows graphs of the dose-dependant inhibition of the binding of labeled cholera toxin to GM1 ganglioside microarrays by unlabeled cholera toxin. The arrays were treated with solutions containing FITC-CTx (1 nM) and unlabeled cholera toxin at different concentrations (0 nM - 200 nM).

Figure 6 shows the dose response of GM1-DLPC microarrays to FITC-labeled cholera toxin.

Figure 7 shows the specific competitive binding of unlabeled cholera toxin (CT, domain B) to the GM1-DLPC arrays against the fluoresently labeled CT.

Figure 8 shows the binding selectivity of fluorescently labeled FITC-CT and FITC-TTx to gangliosides in multiplexed arrays.

The file of this patent contains at least one drawing executed in color. Copies of this patent with color drawing(s) can be obtained from the Patent and Trademark Office upon request and payment of the necessary fee.

## DETAILED DESCRIPTION OF THE INVENTION

Microarrays containing components of cell membranes provide an attractive platform for efficient monitoring and study of fundamental aspects of molecular recognition involved in protein binding at the surface of a cell. Detection of chemical or bacterial toxins and the testing of compounds as potential inhibitors can be a particularly significant and topical application of membrane microarrays. Microarrays have spatially indexed microspots, comprising immobilized "probe" molecules of biological interest. When exposed to an assay sample of interest, "target" molecules in the sample bind to the probe molecules of the microspots to an extent determined by the concentration of the target molecule and its affinity for a particular probe molecule. In principle, if the target concentrations are known, the affinity of the target for the different probe microspots can be estimated simultaneously. Conversely, in principle, given the known affinities of the different molecules in the target for each probe microspot, the amounts of binding observed at each microspot may be used to estimate simultaneously the concentrations of multiple analytes in the sample. The attractiveness of microarray technology lies in the ability to obtain highly multiplexed information using small amounts of sample.

In one embodiment, the present invention describes the fabrication of microarrays having a variety of ion channels integrated in lipid membranes. The ion channels include sodium channel, potassium channel, calcium channel, acetylcholine receptor, ryanodine receptor, glutamate receptor, and any given combinations of these ion channels. These ion channels can be in the form of membrane preparations that are separated from certain cell lines, or in the form of re-folded protein in liposomes, bacteria expressing systems, or the like. The re-folded ion channels can be made using state-of-the-art methods. This type of microarray is preferably used to identify animal toxins and to screen compounds that interfere with the binding of toxins to these ion channels.

In an additional embodiment, a microarray may consist of different lipid compositions. The lipid compositions may include a synthetic lipid such as DLPC (dilaurylphosphatidylcholine), a mixture of different synthetic lipids such as dipalmitoylphosphatidylcholine (DPPC)/dimyristylphosphtidylcholine (DMPC), egg phosphatidylcholine (egg PC), a synthetic host lipid doped with toxin-binding receptors, or a purified toxin-binding receptor. The host lipid can be any given lipid such as a synthetic lipid or a natural lipid (e.g., egg PC). The toxin-binding receptor may include a ganglioside, such as GM1 and GT1b, or a ceramide such as Gal(β)-ceramide, or a cholesterol, or a cerebroside. This type of microarray is preferably used to identify bacterial toxins and screen compounds that interfere with the binding of toxins to these biological membranes. Table 1 presents receptors for bacterial toxins.

Table 1.

| Receptors for Bacterial Toxins | |
|---|---|
| Toxin | Ligands |
| Cholera toxin | GM1: gal($\beta$1,3)GalNAc($\beta$1,4)(NeuAc($\alpha$2,3))Gal($\beta$1,4)Glc($\beta$)-ceramide |
| Heat-labile toxin | GM1 |
| Tetanus toxin | GT1b: gal($\beta$1,3)GalNAc($\beta$1,4)((NeuAc($\alpha$2,8))NeuAc(2,3)Gal($\beta$1,4)Glc($\beta$)-ceramide |
| Botulinum toxin A & E | GD1b: NeuAc($\alpha$2,8)gal($\beta$1,3)GalNAc($\beta$1,4)(NeuAc($\alpha$2,8))NeuAc(2,3)Gal($\beta$1,4)Glc($\beta$)-ceramide |
| Botulinum toxin B | Gal($\beta$)-ceramide |
| Botulinum toxin B, C, F | GT1b |
| Delta toxin | GM2: galNAc($\beta$1,4)(NeuAc($\alpha$2,3))Gal($\beta$1,4)Glc($\beta$)-ceramide |
| Toxin A | Gal($\alpha$1,3)Gal($\beta$1,4)GlcNAc($\beta$1,3)Gal($\beta$1,4)Glc($\beta$)-ceramide |
| Shiga toxin | Gal($\alpha$1,4)Gal($\beta$)-ceramide |
| Vero toxin | Gal($\alpha$1,4)Gal($\beta$1,4)Gac($\beta$)-ceramide |
| Pertussis toxin | NeuAc($\alpha$2,6)Gal |
| Dysenteriae toxin | GlcNAc($\beta$1) |

In another embodiment, a microarray may have a number of biological membranes containing cell surface receptors including G protein-coupled receptors. Preferably expressed, G protein-coupled receptors play key physiological roles in the central neuron system. Preferably, this type of microarray may be used to identify small-molecule chemical toxins (e.g., $\leq$.200-500 kilo-daltons, preferably $\leq$ ~ 2-5 kdaltons).

In yet another embodiment, a microarray of any given combination of these biological membranes can be fabricated and used to screen any given set of toxins. A specific embodiment includes an array of locations of a given biological membrane containing a toxin-binding target.

The method for detecting and identifying a toxin in a sample comprises: providing an array having a plurality of biological membranes associated with a surface of a substrate; contacting the array with a solution having a target compound; monitoring for binding activity of at least one biological membrane with said target compound. The biological membranes are arranged preferably in distinct microspots. The biological membranes contain a toxin-binding moiety, such as a cell-surface protein. The toxin-binding moiety may be a natural lipid, a synthetic lipid, or a lipid composition containing a toxin-binding receptor, or a purified receptor. The toxin-binding moiety may be an G protein-coupled receptor, or an ion channel, such as a sodium channel, a potassium channel, a calcium channel, and any combination of ion channels. The toxin-binding moiety also may be an acetylcholine receptor, a ryanodine receptor, a glutamate receptor, a ceramide, a ganglioside, a cerebroside, sulfatides or cholesterol.

The method employs a labeled toxin or known compounds with an affinity to the toxin molecule or to the receptor site. The target compound can have at least one constituent that is labeled or no labeled constituent. When the constituent is labeled, the monitoring step comprises detecting for the presence of the label. When the constituent is not labeled, the monitoring step comprises detecting directly a physical change due to the binding of said target compound to said biological membranes.

In a second aspect, the invention includes an array for identifying and detecting a toxin. The array comprising a plurality of biological membrane probes associated with a surface of a substrate; said biological membrane containing a toxin-binding moiety or toxin-binding receptor. The toxin-binding receptor is a natural lipid, a synthetic lipid, a lipid composition containing toxin-binding receptor, or a purified receptor. The toxin-binding receptor is a G protein-coupled receptor, a sodium channel, a potassium channel, a calcium channel, an acetylcholine receptor, a ryanodine receptor, a glutamate receptor, a ceramide, a ganglioside, a cerebroside, sulfatides or cholesterol.

The substrate includes glass, metal-oxides, metal, non-metals, silicon, or plastic, which may be configured as a chip, a slide or a microplate. The substrate could be smooth and flat, or porous, with particle or pores on the scale of nanometers or microns (e.g., ~ 1-50 nm up to ~25 or 50-500 $\mu$m). The surface may be coated with a material such as a silane, thiol, disulfide, or a polymer. When the substrate is gold, the coating may be a thiol or disulfide. The silane presents terminal polar moieties, such as hydroxyl, carboxyl, phosphate, sulfonate, thiol, or amino groups. The polymer is a polyamine, such as poly-lysine or a polyethylenenimine. The surface may be positively charged and contains amino groups, such as $\gamma$-aminopropylsilane.

The biological membrane microarrays may be used to identify and detect the presence of toxins in a sample. In this type of application, the specific binding pattern of a given toxin to a set of different biological membrane probes in the microarray is used to identify and detect the presence of a toxin in the sample. The binding of toxins to the biological membrane array can be monitored using fluorescently labeled toxins in combination with state-of-the-art fluorescence detection methods. Alternatively, the binding of toxin to the biological membrane array can be monitored using label-free detection methods such as surface plasmon resonance (SPR) or other optical or electrochemical methods. A biological membrane microarray can also be employed to screen compounds that can interfere with the binding of a given toxin or a given set of toxins to the receptors in the array.

The method for identifying and detecting a toxin in a sample may comprise: providing an array having a plurality of biological membrane microspots associated with a surface of a substrate; contacting a sample solution comprising an unknown toxin with an array of probe biological membrane microspots; and detecting the binding profile of the unknown toxin to at least one or more of the probe microspots. The sample is a biofluid from a specific infectious tissue, or a solution from food or environmental sources. In another embodiment, the sample is an aqueous solution having chemical toxins collected and/or concentrated, for instance in a solution fluid bath, from ambient samples such as contaminated air or other gaseous media.

Examples

Background

As an example, a microarray according to the present invention may have lipids containing the GM1 and GT1b gangliosides. The description that follows demonstrates their use for detecting toxins and screening of compounds as potential toxin inhibitors.

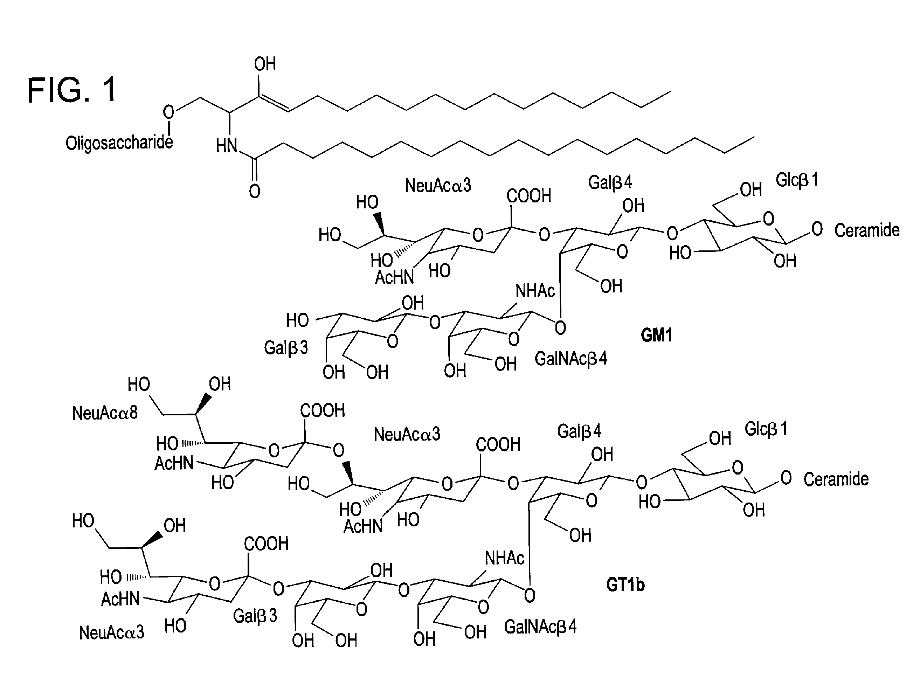

One of the best-studied examples of toxin-ganglioside interactions is the binding of the (cholera) toxin produced by *vibrio cholerae* to the ganglioside GM1. The GM1 ganglioside contains a pentasaccharide (Figure 1) consisting of Gal($\beta$1-3)GalNAc($\beta$1-4)(NeuAc($\alpha$2-3))Gal($\beta$1-4)Glc($\beta$1-1)-ceramide. Studies suggest that the binding epitope of GM1 for the cholera toxin includes the internal Gal and almost all of the external Gal$\beta$3, NeuAc$\alpha$3, and possibly the methyl moiety of the acetamido group of GalNAc$\beta$4. The binding domain of the cholera toxin itself consists of a pentamer of B domains; multivalent interactions with several GM1 groups lead to significantly enhanced affinity of the toxin for the cell surface. The specificity of toxin-carbohydrate interactions is well demonstrated by differences in the binding epitopes between the tetanus and cholera toxins. The tetanus toxin (produced by *clostridium tetani)* binds specifically to the ganglioside GT1b; this ganglioside (NeuAc($\alpha$2-3)Gal($\beta$1-3)GalNac($\beta$1-4)(NeuAc($\alpha$2-8)NeuAc($\alpha$2-3))Gal($\beta$1-4) Glc($\beta$1-1)Ceramide) (Figure 1) contains two sialic acid (NeuAc$\alpha$3 and NeuAc$\alpha$8) residues appended to the GM 1 ganglioside. Binding studies have shown that the Gal$\beta$3GalNAc$\beta$4NeuAc$\alpha$8NeuAc$\alpha$3Gal$\beta$4 moiety is involved in binding to the Hc fragment of the tetanus toxin; unlike the cholera toxin, each toxin contains one such fragment. The He fragment is involved in interactions with the terminal NeuAc$\alpha$8 residue; the lack of this sugar in the GM1 ganglioside explains the reduced affinity of the tetanus toxin for GM1. Conversely, extensions to the terminal Gal$\beta$3 residue of GM1 are not favorable for binding to the cholera toxin; therefore, the additional sialic acid (NeuAc$\alpha$3) in GT1b results in poor binding of the cholera toxin to the GT1b ganglioside.

Fundamentally different from DNA or conventional protein microarrays, membrane microarrays offer exciting possibilities for biopharmaceutical research. A membrane microarray requires the immobilization of the probe molecules of interest and the associated lipids. Another unique aspect of membrane microarrays is the need to keep the probe confined to the microspot while maintaining the desired lateral movement of individual molecules within the microspot - properties that are contradictory and preclude covalent immobilization of the membrane. Assays for microarrays require incubation with different reagents and buffers, and withdrawal through air-water interfaces between these multiple steps; moreover, conventional microarray scanners are not well suited to scanning slides that are wet. Given these considerations, an ideal surface for membrane microarrays should have properties such that: (i) supported membranes on the surface resist physical desorption when withdrawn through air-water interfaces, and (ii) supported membranes on the surface exhibit long-range lateral fluidity.

To test the stability of membrane microarrays, we subjected slides with printed membrane microspots (doped with fluorescently labeled lipids) to repeated immersions into buffer and withdrawl through the buffer-air interface and examined the slides by fluorescence microscopy. The lateral fluidity of supported lipids was tested by traditional fluorescence recovery after photobleaching experiments. Screening of several surfaces revealed that those derivatized with $\gamma$-aminopropylsilane (GAPS) provided the best balance of these properties - microarrays on GAPS resisted desorption (independent of the phase of the lipids) and supported membranes on GAPS exhibited lateral fluidity (with a mobile fraction of ~ 0.5). Furthermore, microarrays of G protein-coupled receptors on GAPS were shown to bind ligands with affinities and specificity consistent with the literature, which demonstrated the feasibility of fabricating membrane protein microarrays.

Materials

Microarrays of lipids containing gangliosides on surfaces coated with γ-aminopropylsilane (GAPS) are fabricated and used for detecting the binding of toxins by fluorescence imaging. The materials used included in one example, 1,2-Dilauroylsn-glycero-3-phosphocholine (DLPC) from Avanti Polar Lipids Inc. (Alabaster, AL). In another example, the materials were dilaurylphosphatidylcholine (DLPC), egg phosphatidylcholine (egg PC), gangliosides (Monosialoganglioside (GM1) and trisialoganglioside (GT1b)), and cholesterol from Sigma Chemical (St. Louis, MO). The toxins included bungarotoxin, cholera toxin B domain (CTx) and FITC-labeled cholera toxin B domain (FITC-CTx) from Sigma Chemical, tetanus toxin fragment C (TTx) and FITC-labeled tetanus toxin fragment C (FITC-TTx) from Calbiochem (Pasadena, CA). All toxins were handled with extreme care; a 2M sodium hydroxide solution or bleach was used for decontamination. GAPS slides from Corning Inc. (Corning, NY) were used.

Using a quill-pin printer (Cartesian Technologies Model PS 5000) equipped with software for programmable aspiration and dispensing features, lipid microarrays can be deposited in a microplate, or GAPS coated slides. Before printing, thin films of dilaurylphosphatidylcholine (DLPC) (1mg/ml) or egg phosphatidylcholine (egg PC) lipid in the absence or presence of 4 mol% ganglioside (GM1 or GT1b) in 20 mM phosphate buffer (pH 7.4), were sonicated to clarity to form small vesicles of lipids. For printing, 25 μL of each lipid solution was added to different wells of a 384 well microplate. A single insertion of the pin into the solution yielded 3 identical spots within a single array. To prevent contamination due to carry-over between different lipid solutions, an automatic wash cycle was incorporated that consisted of consecutive washes of the pin in ethanol and water. After printing, the arrays were incubated in a humid chamber at room temperature for one hour to enable possible lateral redistribution of lipid molecules in the supported membrane, and then used for detecting the binding of toxins. For the binding assays, each individual array was incubated with 20 μl of a solution containing labeled toxin in the absence and presence of varying amounts of unlabeled toxin. The binding buffer used for all experiments was 20 mM phosphate buffer, pH 7.4, 0.2% BSA.

Toxin Binding Assays.

The binding assays were carried out by incubating arrays of the gangliosides with the appropriate solution, washing with buffer to remove unbound toxins (and potential inhibitors), and scanning using a fluorescence scanner. The binding assays were designed to test: (I) the selectivity of binding; (II) the specificity of inhibition; and (III) the dose dependency of binding and inhibition.

*(I) Selectivity of Binding.*

Microarrays are naturally suited for simultaneously screening the binding of a compound to multiple probes. In a typical primary screening experiment, the array is incubated with a compound at a particular concentration; if a positive signal (a "hit") is obtained for a probe microspot, more detailed analysis (e.g. dose dependency studies described below) is carried out. Our studies were aimed at simply establishing the specificity of binding to ganglioside microarrays using known toxin-ganglioside interactions.

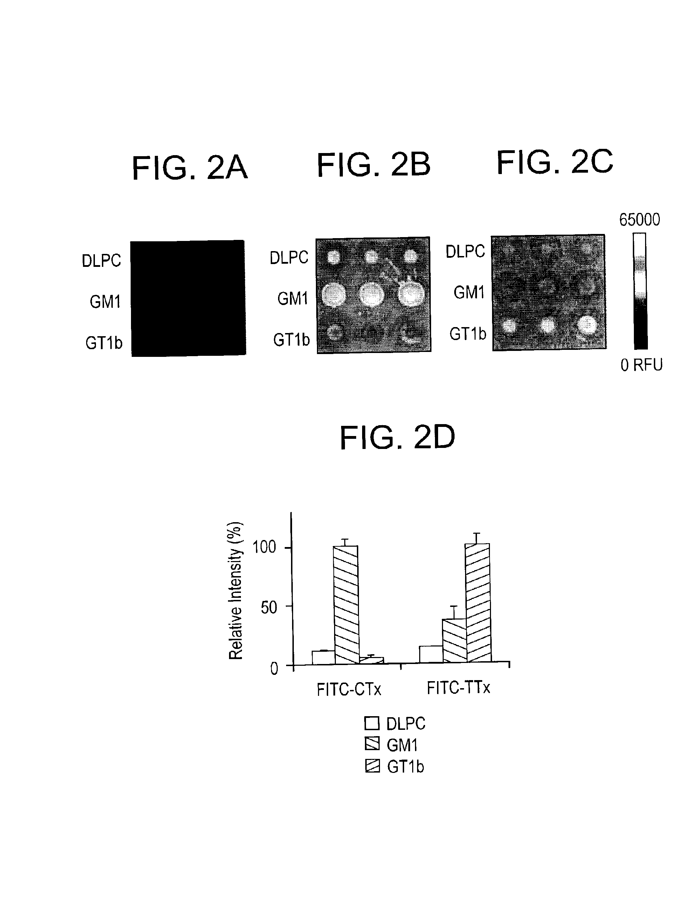

Figures 2A-C show fluorescence false color images of three identical microarrays on a single GAPS slide; each microarray consists of three replicate microspots of DLPC (top row), DLPC doped with GM1 (middle row), and DLPC doped with GT1b (bottom row). The first microarray was treated with buffer only and serves as a negative control. As expected, no signal is observed on any of the microspots in Figure 2A. The second microarray in Figure 2B was incubated with a solution of fluorescently labeled cholera toxin (B domain; FITC-CTx). Strong binding to microspots containing the GM1 ganglioside is observed, as expected, although weak binding (<10% of the signal obtained with GM1) to microspots of DLPC and DLPC doped with GT1b is also observed. When the microarray was treated with a solution containing fluorescently labeled tetanus toxin (C fragment; FITC-TTx) in Figure 2C, the highest amount of binding was found to correspond to microspots containing the GT1b ganglioside, in accordance with the known specificity of the toxin. The binding signal is lower than that observed for binding of FITC-CTx to GT1b; sub-optimal amounts of GT1b in the mixed lipid or issues with labeling of the tetanus toxin could be possible reasons for the poorer signal. In Figure 2D, the signal observed for binding of FITC-TTx to the GM1 microspots is approximately 35% of that observed for binding to GT1b microspots. Previous researchers have reported that the tetanus toxin does not bind to GM1; it is hypothesize that this binding phenomenon is non-specific in nature and appears accentuated due to the inherently low net signal (signal minus background) (~ 3000 RFU) for binding of FITC-TTx to GT1b.

(II) *Selectivity of Inhibition.*

Since labeling of molecules with fluorescent dyes is tricky, compounds are more commonly screened using competition assays in which the unlabeled compound is allowed to compete for probe binding sites with a cognate, labeled ligand for that probe. The decrease in fluorescence relative to an array incubated only with the labeled ligand provides a measure of inhibition by the unlabeled compound. In a typical initial screen, the unlabeled compound is present in large excess (e.g. 100-fold) and a significant (e.g. >50%) decrease in fluorescence leads to further investigation of that compound.

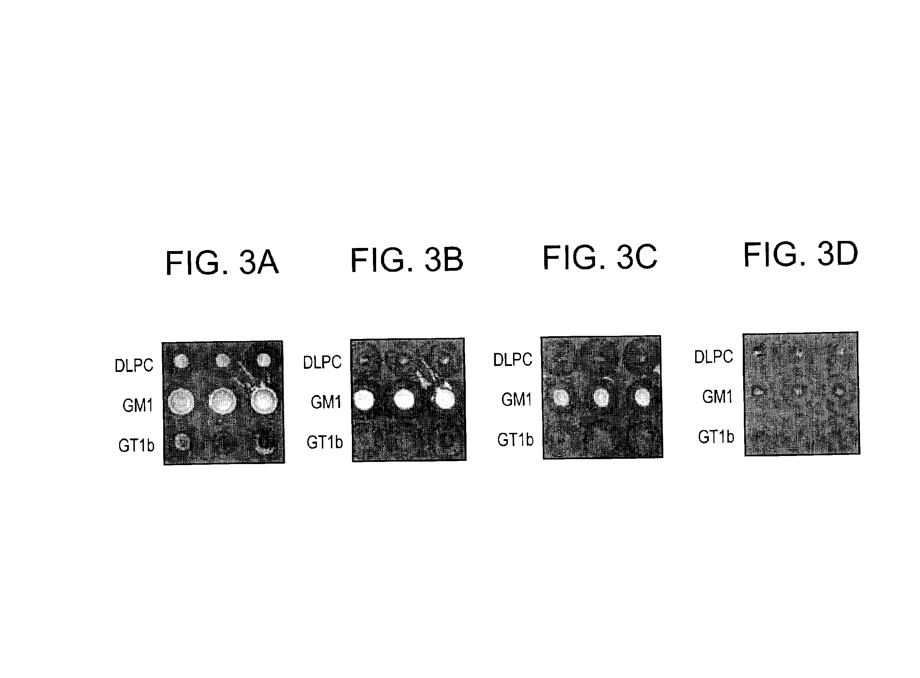

Figures 3A-D show fluorescence images of four microarrays (identical in probe content to that used in Figure 2), which were treated with solutions containing FITC-CTx and either the tetanus toxin, bungaratoxin or unlabeled cholera toxin. Figure 3A, in which the array was treated with fluorescently labeled cholera toxin, provides the reference for measuring inhibition. Figure 3B is a fluorescence image of an array treated with a mixture of the labeled cholera toxin (1 nM) and unlabeled tetanus toxin (100 nM). There is a small but insignificant decrease in the amount of binding to the GM1 ganglioside microspots; interestingly, the small amount of binding of FITC-CTx to microspots of the GT1b toxin (see Figure 2B) is completely inhibited. The presence of bungaratoxin also causes no significant decrease in the amount of binding of FITC-CTx (Figure 3C). When the microarray was incubated with a mixture containing excess unlabeled cholera toxin (100 nM), there was essentially complete inhibition of binding of FITC-CTx to the GM1 microspots (Figure 3D). In a corresponding series of experiments, we observed specific inhibition of FITC-TTx binding by the unlabeled tetanus toxin (data not shown). These experiments demonstrate the feasibility of screening potential inhibitors against toxins using ganglioside microarrays.

(III) *Dose Dependency of Binding and Inhibition.*

One of the questions that arises in the development of a microarray based assay (or any solid phase assay) is whether the affinity between two compounds changes upon immobilization of one of the binding partners (as a probe). For microarrays, the estimation of binding constants requires direct comparisons of the binding signals between microspots treated with different concentrations of an analyte. This comparison is not straightforward as it often requires measurements of small differences in fluorescence and demands much greater precision than that required for screening applications.

For the microarrays described herein, the probe (GM1 or GT1b) was mixed with a host lipid (DLPC). For a particular preparation of the DLPC/ganglioside mixture, it is reasonable to assume that the surface mole fraction of ganglioside (and DLPC) is the same from spot to spot. The surface binding capacity for the cholera toxin was estimated to be at a ganglioside density of about 4 mol %. The estimate as outlined below does not take into account inhomogeneities in the distribution of gangliosides in the host lipid. The radius of the pentameric binding domain of the cholera toxin is ~ 3 nm. Assuming a close packed structure, the number of cholera toxin molecules per square micron is ~ 2.3 x $10^4$. Assuming 1:1 binding between GM1 and each domain of the cholera toxin, the number of binding sites corresponding to a monolayer of the cholera toxin is ~1.2 x $10^5$/$\mu m^2$. Using Langmuir-Blodgett techniques, the area occupied by a GM1 molecule in a GM1 monolayer at the air water interface was estimated to be ~ 0.6 $nm^2$. Assuming an area of ~ 0.5 $nm^2$ per DLPC molecule, we estimate that there are ~ 2 x$10^6$ molecules of DLPC and ~ 8 x$10^4$ molecules of GM1, per $\mu m^2$. The number of GM1 molecules (at ~ 4 mol %) per unit area is approximately half the number of binding sites required for binding a full monolayer of the cholera toxin. In reality, given the nanomolar affinity of the cholera toxin for ganglioside presenting surfaces, binding of the toxin is essentially irreversible. Hence, assuming a random sequential adsorption model in which approximately 55% coverage is maximally possible, the number of cholera toxin molecules that can bind to a surface presenting GM1 molecules is ~ 6.3 x $10^4$ molecules per $\mu m^2$. Therefore, a DLPC surface doped with ~ 4 mol % GM1 is likely to be sufficient to support the maximum possible amount of binding of the cholera toxin.

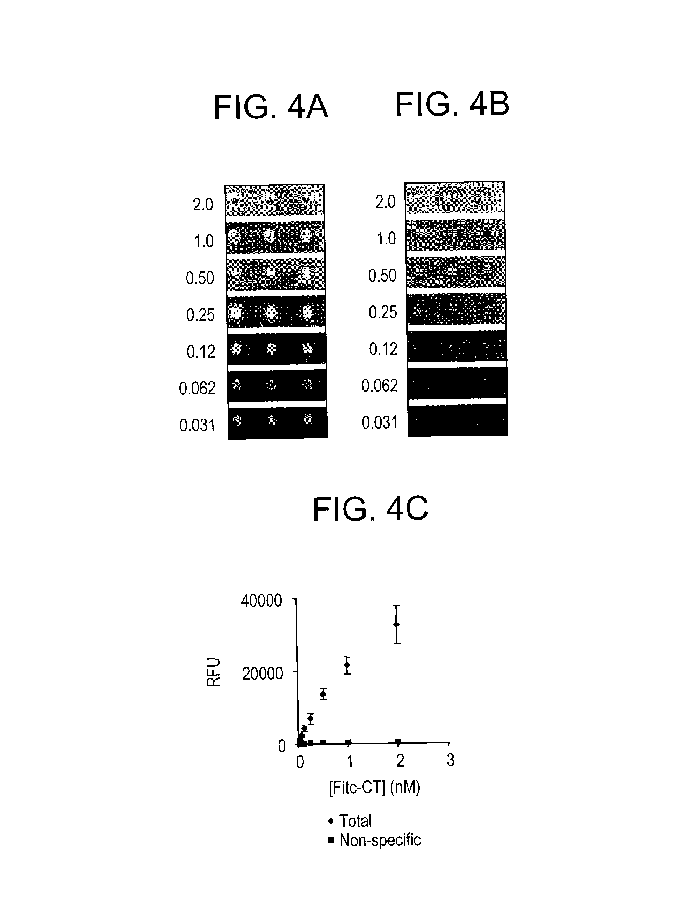

Figure 4 shows fluorescence images of microarrays of the GT1b ganglioside (4 mol %) treated with different concentrations of FITC-CTx. The signal is dependent on the concentration of FITC-CTx. To estimate the amount of non-specific binding, a corresponding set of microarrays was treated with a mixture containing FITC-CTx at the same concentrations and excess unlabeled cholera toxin. The difference between the signals at each concentration of FITC-CTx provides a measure of the amount of specific binding to the GM1 microspots. Specific binding is observed even at a concentration of ~ 30 pM, which demonstrates the excellent sensitivity of the fluorescence assay. The amount of binding is linear at concentrations less than 1 nM. Unfortunately, the background signals at higher concentrations of FITC-CTx are too high to observe saturation of the binding signal.

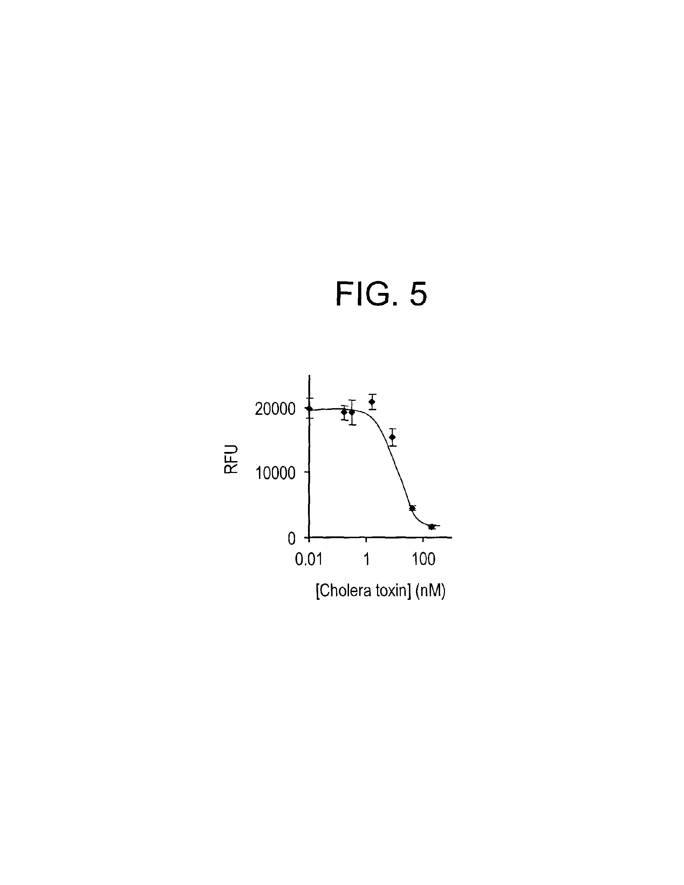

The binding of the cholera toxin to gangliosides is multivalent. The binding affinity is dependent on the valence of the interaction, hence the binding cannot be characterized by a single dissociation constant. The estimated binding constant below represents the "average" affinity of the ganglioside microspots for the cholera toxin. To estimate this affinity, in a competition assay, GM1 microarrays were treated with increasing concentrations of unlabeled cholera toxin at a fixed concentration of FITC-CTx (1 nM). From the inhibition profile as shown in Figure 5, $IC_{50}$ is estimated to be ~ 20 nM. The estimate of $K_i$ from the measured $IC_{50}$ value is given by Equation 1, where $K$; is the equilibrium dissociation constant for the inhibitor, L is the concentration of FITC-CTx, and $K_L$ is the equilibrium dissociation constant of FITC-CTx.

$$K_i = \frac{IC_{50}}{1 + \frac{L}{K_L}} \tag{1}$$

Using the present method one can estimate the binding affinity. Although values for the binding constant between the cholera toxin and the GT1b ganglioside as reported in the scientific literature have significant discrepancies, a reasonable "consensus" value is ~ 2 nM. Even though difficult to estimate directly the value of $K_L$, it is reasonable to used $K_L$ = 2 nM, and assume that labeling of the toxin does not influence its binding affinity. Based on such assumptions, the value was estimated to be $K_i$ ~ 13 nM; this "average" affinity is ~ 25-times greater than the affinity of the toxin for the soluble GM1 pentasaccharide. The relative contributions of polyvalency and the presentation of GM1 as a ganglioside (as opposed to a free sugar) are presently unclear. The data demonstrate the feasibility of estimating affinities of potential inhibitors using ganglioside microarrays.

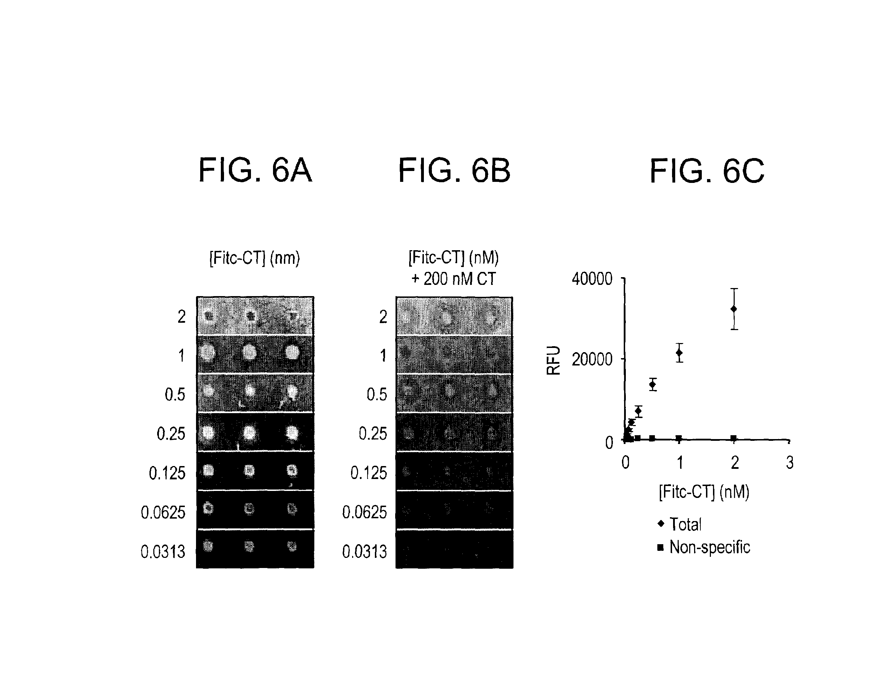

The results of specific binding of cholera and tetanus toxins to microspots containing GM1 and GT1b gangliosides, respectively, suggest that the possibility of using ganglioside microarrays for toxin identification and the screening of compounds that can inhibit toxin binding. The main results were presented in Figure 6, 7 and 8. Figure 6 shows the dose response of fluorescently labeled toxin (Fitc-cholera toxin B, Fitc-CT) binding to a microarray of DLPC doped with 4% gangloside GM1 on a GAPS slide. On the left are fluorescence images of the microarray after Fitc-CT binding. On the right is a plot of fluorescence intensity of DLPC/GM1 arrays as a function of the concentration of Fitc-CT in the absence (the total) and presence (non-specific) of excess unlabeled cholera toxin B.

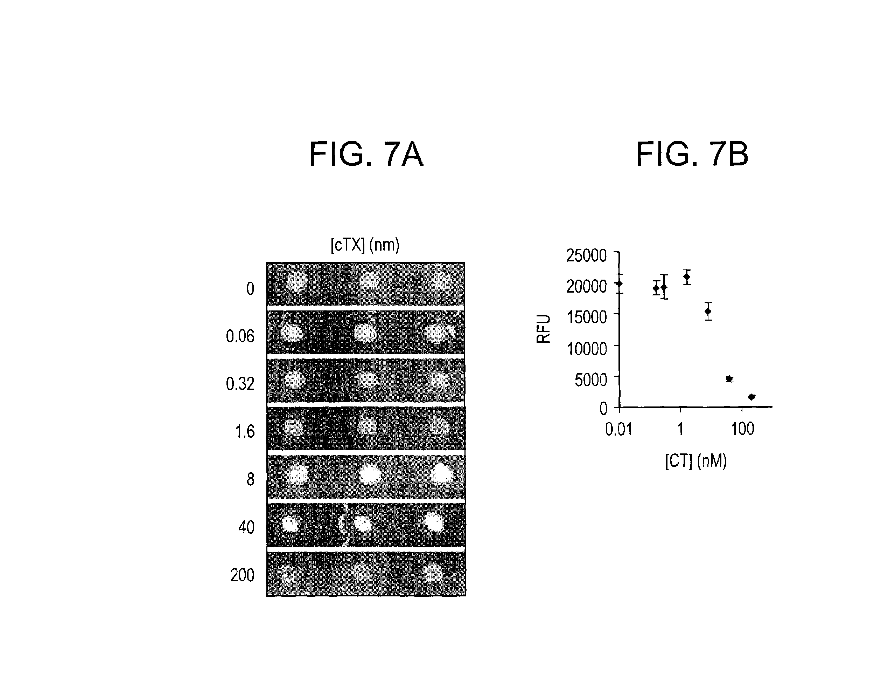

Figure 7 shows the specific competitive binding of unlabeled cholera toxin B with fluorescently labeled FITC-CT to the array of DPLC-doped with 4% ganglioside GM on a GAPS slide. On the left is a fluorescence image of DPLC/GM1 mmicroarrays after the binding of 1 nM Fitc-CT in the presence of increasing concentration of cholera toxin B (from top to bottom). On the right is an image of fluorescence intensity of the array of DLPC/GM1 after the binding of Fitc-CT as a function of the concentration of unlabeled CT. These results suggest that CT specifically binds to the GM1.

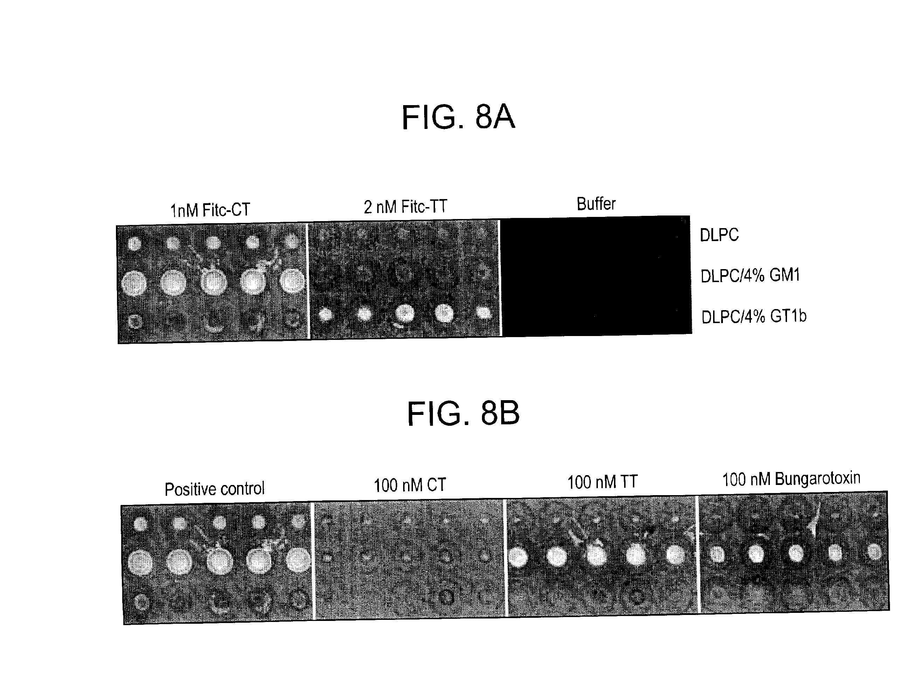

Figure 8 shows the binding selectivity of fluorescently labeled FITC-CT and FITC-tetanus toxin to gangliosides in a multiplexed arrays. These results suggest that FITC-CT specifically binds to GM1, and FITC-tetanus toxin preferably binds to GT1b in the arrays. In Figure 8A show fluorescence images of three multiplexed microarray after the incubation with three different solutions: 1 nM Fitc-CT, 2 nM FITC-Tetanus toxin fragment C (FITC-TT), and buffer only, respectively. Each microarray consists of three rows of different lipid compositions. From top to bottom, there are DLPC alone, DLPC doped with 4% GM1, and DPLC doped with 4% GT1b, respectively. As shown by the fluorescence intensity, FITC-CT specifically binds to microsopts of DLPC/GM1, while the FITC-TT preferably binds to microsopts of DLPC/GT1b. Figure 8B shows fluorescence images of four multiplexed microarray after the incubation with 1 nM Fitc-CT in the absence of any unlabeled toxin (I), 100 nM CT (II), 100 nM Tetanus toxin (III), and 100 nM bungarotoxin (IV). As shown by the fluorescence intensity, only unlabeled CT can specifically block the binding of FITC-CT to the DLPC/GM1.

The present invention has been described in detail and by way of examples of preferred embodiments. Persons in the art, however, can appreciate that substitutions, modifications, and variations may be made to the present invention and its uses without departing from the scope of the invention, as defined by the appended claims and their equivalents.

## Claims

1. A method for detecting a binding event between a probe and target compound, said method comprising: providing an array having a plurality of biological membrane probes associated with a surface of a substrate; contacting a sample having a target compound with said array of biological membrane probes; and detecting or monitoring a binding event between at least one or more of the probes with one or more of the constituents of the target compound.

2. A device for detecting and identifying a toxin, the device comprises: an array with a plurality of biological membrane probes associated with a surface of a substrate; said biological membrane containing a toxin-binding moiety.

3. The method according to claim 1, wherein the array of microspots is incubated with labeled target and an unlabeled target compound, and the binding event between the unlabeled target compound and the probe is determined by measuring a decrease in the signal of the label due to competition between the labeled target and the unlabeled target compound for the probe.

4. The method according to claim 1, wherein said sample comprises an unknown toxin with said array of biological membrane microspots; and detecting the binding profile of the unknown toxin to at least one or more of the microspots.

5. The method according to claim 1, wherein the sample is a biofluid from a specific infectious tissue, a solution from food or environmental sources, or an aqueous solution having chemical toxins collected or concentrated from a contaminated gaseous media.

6. The method according to claim 1, wherein said biological membranes contain a toxin-binding moiety.

7. The invention according to either claim 1 or 2, wherein said biological membranes are arranged in distinct microspots.

8. The invention according to any one of claims 1-7, wherein said toxin-binding moiety is a cell-surface protein.

9. The invention according to any one of claims 1-8, wherein the toxin-binding moiety is a natural lipid, a synthetic lipid, or a lipid composition containing a toxin-binding receptor, or a purified receptor.

10. The invention according to any one of claims 1-8, wherein the toxin-binding receptor is a sodium channel, a potassium channel, a calcium channel, or any combination of ion channels, an acetylcholine receptor, a ryanodine receptor, a glutamate receptor, a G protein-coupled receptor, a ceramide, a ganglioside, a cerebroside, sulfatides or cholesterol.

11. The invention according to any one of claims 1 and 7-10, wherein the target compound has at least one constituent that is labeled, and the detection or monitoring of the label is carried out by imaging based on fluorescence, phosphorescence, chemiluminescence, or resonance light scattering emanating from the bound target.

12. The invention according to any one of claims 1 and 7-10, wherein the target compound is unlabeled and the binding event is determined by a change in physical properties at the interface.

13. The invention according to either claims 1 or 12, wherein the monitoring step includes detecting directly a physical or chemical change due to the binding of said target compound to said biological membranes.

14. The invention according to claim 1, wherein said method employs a labeled toxin or known compounds with an affinity to the toxin molecule or to the receptor site.

15. The invention according to any one of claims 1 and 4-14, said toxin sample can be a synthetic or natural toxin, or from a human, animal, plant, food, or environmental source.

16. The invention according to any one of claims 1-15, wherein the substrate comprises glass, ceramic, metal-oxide, metal, non-metal, silicon, or polymer.

17. The invention according to any one of claims 1-16, wherein the substrate is either nano- or micro-porous.

18. The invention according to any one of claims 1-17, wherein the surface is coated with a material, including one of the following: a silane, thiol, disulfide, or a polymer; terminal polar moieties having a hydroxyl, carboxyl, phosphate, sulfonate, thiol, or amino groups; or a γ-aminopropylsilane, poly-lysine, polyethyleneimine, or chitosan.

19. The invention according to any one of claims 1-18, wherein the surface is positively charged.

20. The invention according to any one of claims 1 and 3-6, wherein said-toxin binding moiety is a carbohydrate.

FIG. 1

GM1

GT1b

EP 1 376 124 A2

FIG. 2A    FIG. 2B    FIG. 2C

FIG. 2D

EP 1 376 124 A2

FIG. 3A   FIG. 3B   FIG. 3C   FIG. 3D

DLPC

GM1

GT1b

# FIG. 4A    FIG. 4B

# FIG. 4C

# FIG. 5

# FIG. 6A

# FIG. 6B

# FIG. 6C

[Fitc-CT] (nm)

[Fitc-CT] (nM)
+ 200 nM CT

# FIG. 7A

# FIG. 7B

[cTX] (nm)

EP 1 376 124 A2

# FIG. 8A

# FIG. 8B